(19) **Europäisches Patentamt**
**European Patent Office**
**Office européen des brevets**

(11) **EP 3 033 997 B1**

(12) **EUROPÄISCHE PATENTSCHRIFT**

(45) Veröffentlichungstag und Bekanntmachung des Hinweises auf die Patenterteilung:
**09.09.2020  Patentblatt 2020/37**

(51) Int Cl.:
*A61B 5/06* ^(2006.01)   *A61B 5/00* ^(2006.01)
*A61B 34/20* ^(2016.01)   *A61B 1/00* ^(2006.01)
*A61B 1/005* ^(2006.01)   *A61B 1/04* ^(2006.01)
*A61B 1/06* ^(2006.01)

(21) Anmeldenummer: **14198758.6**

(22) Anmeldetag: **18.12.2014**

(54) **Endoskopsystem zur Bestimmung einer Position und einer Orientierung eines Endoskops in einem Hohlraum**

Endsocope system for determining a position and an orientation of an endoscope within a cavity

Système d'endoscope pour la détermination d'une position et d'une orientation d'un endoscope dans un espace creux

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.06.2016  Patentblatt 2016/25**

(60) Teilanmeldung:
**16163497.7 / 3 056 145**

(73) Patentinhaber: **Karl Storz SE & Co. KG**
**78532 Tuttlingen (DE)**

(72) Erfinder:
• **Schwarz, Peter**
  **78532 Tuttlingen (DE)**
• **Irion, Klaus M.**
  **78532 Tuttlingen (DE)**
• **Fallert, Johannes**
  **78532 Tuttlingen (DE)**
• **Lowag, Florus**
  **78532 Tuttlingen (DE)**

(56) Entgegenhaltungen:
JP-A- 2014 151 102    JP-A- 2014 230 658
US-A- 6 122 538      US-A1- 2011 032 347

EP 3 033 997 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft ein Endoskopsystem, ausgebildet für ein Verfahren zur Bestimmung einer Position und einer Orientierung eines Endoskops in einem Hohlraum.

Viele medizinische Untersuchungen und chirurgische Operationen werden heute endoskopisch durchgeführt. Hierdurch kann die Belastung des Patienten erheblich verringert werden. Aufgrund des durch den endoskopischen Zugang eingeschränkten Blickfeldes erfordern endoskopische Operationen jedoch ein erhebliches Maß an Übung des Operateurs, um einen endoskopischen Eingriff effizient und sicher durchzuführen. Insbesondere kann die durch das eingeschränkte Blickfeld eines Endoskops erschwerte Orientierung des Operateurs in einem körperinneren Hohlraum dazu führen, dass bei einer endoskopischen Untersuchung Bereiche der Oberfläche des Hohlraums übersehen werden, d.h. nicht endoskopisch erfasst werden.

Es sind deshalb Systeme entwickelt worden, die einem Anwender eines Endoskops die Orientierung innerhalb des Hohlraums erleichtern. Derartige Systeme, die im vorliegenden Zusammenhang auch als "Navigationssysteme" bezeichnet werden, dienen zur Unterstützung des Anwenders bei der Navigation in dem Hohlraum, wobei die Position und/oder die Orientierung des Endoskops erfasst wird. So sind elektromagnetische Tracking-Systeme bekannt, die ein externes Magnetfeld erzeugen und durch an dem Endoskop angeordnete elektromagnetische Sensoren die Erfassung der Position und der Orientierung des Endoskops in dem externen Magnetfeld ermöglichen. Ferner sind optische Tracking-Systeme bekannt, bei denen die Position eines am proximalen, d.h. benutzernahen, Ende des Endoskops angeordneten Referenzkörpers mit optischen Verfahren bestimmt wird. Mit derartigen Navigationssystemen können die Position und die Orientierung des Endoskops im Raum bestimmt werden, d.h. in drei Rotationsfreiheitsgraden und in drei Translationsfreiheitsgraden durch Ermittlung von drei Winkeln und drei Koordinaten relativ zu einem extrakorporalen Koordinatensystem. Derartige elektromagnetische bzw. optische Tracking-Systeme sind jedoch störanfällig. So erfordert ein optisches System eine stete Sichtverbindung zwischen dem Endoskop und der optischen Sensorik, die beispielsweise eine Stereokamera sein kann. Bei einem elektromagnetischen System können metallische Gegenstände zu einer Störung des für die Verfolgung ("Tracking") des Endoskops notwendigen Magnetfelds führen. Zudem sind optische und elektromagnetische Navigationssysteme mit einem hohen Kostenaufwand verbunden und können außerdem die Arbeit eines Operateurs behindern, beispielsweise durch den Raumbedarf der zur Erzeugung eines Magnetfelds notwendigen Spulen.

Aus US 6,122,538 sind ein System und ein Verfahren zum Verfolgen der Position und der Orientierung einer medizinischen Ultraschallsonde bekannt, die zwei unterschiedliche Sensortypen umfasst. Ein erster Sensor ist ein Neigungssensor zum Messen einer Orientierung der Sonde relativ zur Gravitationsrichtung, und der zweite Sensor ist ein magnetischer Sensor zur Messung einer Orientierung oder einer Position der Sonde relativ zu einem Referenzpunkt. Weiterhin können ein gyroskopischer Sensor und ein Accelerometer vorgesehen sein. Dabei kann eine Rekalibrierung von Sensoren mittels Messungen anderer Sensoren erfolgen.

In US 8,419,717 B2 ist ein medizinisches robotisches System offenbart, das einen robotischen Gelenkarm umfasst, der ein chirurgisches Werkzeug hält, das mittels einer ebenfalls von dem Gelenkarm gehaltenen Werkzeugführung durch eine Inzision in den Körper eines Patienten eingeführt werden kann. Ein Endeffektor weist einen Endpunktsensor auf, der Accelerometer, als MEMS (Micro Electro Mechanical System) ausgebildete Gyroskope und elektromagnetischen Sensoren umfassen kann. Zur Erfassung der Stellung der Gelenke des Gelenkarms können lineare Wegsensoren vorgesehen sein.

In US 2011/0046637 A1 ist ein medizinisches Instrument beschrieben, das zur Messung von Kräften in fünf Freiheitsgraden und einer Lage einer Spitze in sechs Freiheitsgraden ausgebildet ist, um einem Benutzer eine Kraftrückwirkung zu vermitteln. Zur Verfolgung der Position und der Orientierung einer Spitze eines austauschbaren Werkzeugs in sechs Freiheitsgraden können Accelerometer, Gyroskope, elektromagnetische oder optische Tracking-Systeme verwendet werden. Alternativ ist ein Trokar, durch den das Instrument eingeführt wird, mit optischen Sensoren versehen, die über das Erfassen von Markierungen oder der Oberflächentextur auf einem Außenschaft eine Einführtiefe und einen Rollwinkel des Instruments messen.

Gemäß US 2011/0032347 A1 umfasst ein Endoskopiesystem ein Endoskop mit einer an dessen Spitze angeordneten Kamera. Das Endoskop erstreckt sich durch eine Endoskopführung zur Führung und Messung der Bewegung des Endoskops bei der Einführung in den Körper. Ein Bewegungssensor der Führung umfasst einen optischen Sensor und einen Detektor, die entlang eines Durchgangs befestigt sind, um die lineare Bewegung beim Einführen oder Herausziehen des Endoskops und ebenso die von einem Benutzer des Endoskops bewirkte Rotation des Endoskops zu messen. Zur Reduzierung der Mehrdeutigkeit der Daten können Gyroskope an der Spitze des Endoskops verwendet werden, die eine zusätzliche Abschätzung der Position der Kamera liefern.

In JP2014230658 wird ein Endoskopiesystem offenbart, dass eine Kalibriereinheit umfasst, in die das Endoskop eingeführt wird. Dabei enthält diese Kalibriereinheit einen Linear-Encoder während am Endoskop entweder ein Beschleunigungssensor oder ein Rotationssensor angebracht ist. Mit diesen Sensoren wird die Einschubdistanz und die Rotation des Endoskops ermittelt.

In JP2014151102 wird ein Endoskopsystem offenbart, dass eine Einführhilfe umfasst, die die Einführung des Endoskops mit Hilfe von Biege- Beschleunigungs- und Drehratensensoren ermittelt. Zusätzlich sind Magnetsensoren auf dem Endoskop angebracht. Mit Hilfe all dieser Sensoren wird die Lage des Endoskops ermittelt. Navigationssysteme, die auf dem Einsatz von Accelerometern und/oder Gyroskopen beruhen, sind prinzipiell kostengünstiger verfügbar als die zuvor genannten optischen und elektromagnetischen Navigationssysteme. Allerdings können derartige Inertialsensoren nur eine Neigung zur Richtung des Gravitationsfelds sowie Beschleunigungen und Winkelgeschwindigkeiten erfassen. Die Erfassung einer Raumposition und einer Orientierung im Raum ist hiermit nur mit eingeschränkter Genauigkeit möglich.

Es ist Aufgabe der vorliegenden Erfindung, ein Endoskopsystem zur Bestimmung einer Position und einer Orientierung eines Endoskops in einem Hohlraum anzugeben, wobei die oben genannten Nachteile möglichst vermieden werden, insbesondere ein Endoskopsystarn, das eine Bestimmung einer Position und einer Orientierung des Endoskops im Raum mit einer verbesserten Genauigkeit und bei einem geringen Kostenaufwand ermöglicht.

Diese Aufgabe wird durch ein Endoskopsystem gemäß Anspruch 1 gelöst.

Vorteilhafte Weiterbildungen der Erfindung ergeben sich aus den Unteransprüchen.

Ein Verfahren bezieht sich auf ein Endoskop, das einen langerstreckten Schaft umfasst, der zur Einführung in einen Hohlraum, insbesondere einen körperinneren Hohlraum, durch eine Zugangsöffnung ausgebildet ist, wobei die Zugangsöffnung beispielsweise eine natürliche oder eine künstlich geschaffene Körperöffnung sein kann. Das Endoskop weist insbesondere eine Beobachtungsoptik mit einem in einem distalen, d.h. benutzerfemen, Endbereich des Schafts angeordneten Endoskopobjektiv auf, die zur Erzeugung eines Bilds eines Objektfelds in dem körperinneren Hohlraum, d.h. zur Erzeugung eines endoskopischen Bilds, ausgebildet ist.

Das Verfahren bezieht sich weiterhin darauf, dass der Schaft des Endoskops durch eine mit der Zugangsöffnung des Hohlraums verbundene endoskopische Einführvorrichtung in den Hohlraum hineinreicht, d.h. zumindest ein distaler Endbereich des Schafts, insbesondere die Endoskopspitze, ist innerhalb des Hohlraums angeordnet, während ein weiterer Bereich des Schafts sich innerhalb der Zugangsöffnung befindet. Ein proximaler Endbereich des Endoskops, beispielsweise ein Endoskopkopf oder ein Handgriff, sowie ggf. ein proximaler Endabschnitt des Schafts, kann sich außerhalb des Hohlraums und außerhalb der Zugangsöffnung befinden. Insbesondere werden somit die Position und Orientierung der Spitze des Endoskops bestimmt, wobei sich die Spitze in dem Hohlraum befindet und der Schaft des Endoskops durch eine mit der Zugangsöffnung des Hohlraums verbundene Einführvorrichtung verläuft. Vorzugsweise ist der Schaft des Endoskops zuvor durch die Einführvorrichtung von außerhalb eines Körpers in den Hohlraum, der ein körperinnerer Hohlraum sein kann, eingeführt worden. Die endoskopische Einführvorrichtung definiert insbesondere eine Einführöffnung zum Einführen des Endoskops in den Hohlraum. Die endoskopische Einführvorrichtung kann etwa als Trokar bzw. Trokartubus ausgebildet sein, der in eine natürliche oder künstlich geschaffene Körperöffnung einsehbar ist und durch dessen Durchgangsbohrung, die die Einführöffnung darstellt, der Schaft des Endoskops in den Hohlraum eingeführt werden kann.

Erfindungsgemäß werden mit mindestens einem dem Endoskop zugeordneten Beschleunigungssensor (Accelerometer) eine auf das Endoskop einwirkende Beschleunigung und mit mindestens einem dem Endoskop zugeordneten Drehratensensor (Gyroskop) eine Drehbewegung des Endoskops erfasst. Beschleunigungssensoren und Drehratensensoren werden hier auch zusammenfassend als "Inertialsensoren" bezeichnet. Im Folgenden wird unter "Beschleunigung" eine lineare Beschleunigung im Unterschied zu einer Winkelbeschleunigung verstanden, während eine Drehbewegung durch die Winkelgeschwindigkeit charakterisiert wird. Die vom Beschleunigungssensor erfasste Beschleunigung umfasst dabei die Gravitationswirkung ebenso wie eine Beschleunigung, die etwa durch eine von einem Anwender auf das Endoskop ausgeübte Kraft verursacht wird. Die von dem Beschleunigungssensor erfasste Beschleunigung ist insbesondere mit Betrag und Richtung oder, was hierzu gleichwertig ist und rechnerisch umgerechnet werden kann, mit ihren drei Raumkomponenten in drei senkrecht aufeinander stehenden Richtungen erfassbar. Die Drehbewegung ist vorzugsweise als Winkelgeschwindigkeit, die durch Betrag und Richtung oder durch ihre drei Raumkomponenten charakterisiert ist, erfassbar. Der mindestens eine Beschleunigungs- und der mindestens eine Drehratensensor erfassen somit vorzugsweise die räumliche Beschleunigung bzw. die Drehung im Raum, zumindest erfasst der Drehratensensor eine Drehung um eine vertikale Achse. Vorzugsweise sind der mindestens eine Beschleunigungs- und der mindestens eine Drehratensensor innerhalb des Endoskops bzw. innerhalb eines Teilbereichs des Endoskops angeordnet.

Weiterhin wird erfindungsgemäß mit mindestens einem der Einführvorrichtung zugeordneten Einführsensor eine Einführbewegung des Schafts relativ zur Einführvorrichtung erfasst. Der Begriff "Einführbewegung" bezeichnet in der vorliegenden Erfindung die Bewegung des Schafts innerhalb der Einführvorrichtung bzw. einer Einführöffnung, die durch die Einführvorrichtung gebildet wird, und bezeichnet sowohl die Bewegung beim Einführen des Endoskops in den Hohlraum als auch Bewegungen des Schafts relativ zur Einführvorrichtung, die ausgeführt werden, wenn der Schaft in den Hohlraum eingeführt ist. Insbesondere kann hierbei eine Einführlänge bestimmt werden, die die in den Hohlraum eingeführte Länge des Schafts des Endoskops angibt. Ferner kann auch die Bewegung beim Entnehmen des Endoskops aus dem Hohlraum erfasst werden.

Erfindungsgemäß werden eine Position und eine Orientierung des Endoskops, zumindest eines Teilbereichs des Endoskops, insbesondere des distalen Endbereichs des Schafts des Endoskops, aufgrund der erfassten Beschleunigung, Drehbewegung und Einführbewegung ermittelt. Hierfür kann eine Steuerungseinrichtung vorgesehen sein, die zur Erfassung der Signale des mindestens einen Beschleunigungssensors, des mindestens einen Drehratensensors und des mindestens einen Einführsensors mit den betreffenden Sensoren verbunden ist und Prozessormittel zur Ermittlung der Position und Orientierung des Endoskops, zumindest eines Teilbereichs des Endoskops, insbesondere des distalen Endbereichs des Schafts des Endoskops, aus den erfassten Signalen umfasst. Die Steuerungseinrichtung kann weiterhin zur Anzeige der Position und der Orientierung des distalen Endbereichs des Schafts des Endoskops in grafischer oder nummerischer Form eingerichtet sein und hierfür mit einer entsprechenden Anzeigeeinrichtung verbindbar sein oder eine solche umfassen. Insbesondere können auf der Anzeigeeinrichtung nach Art einer 3D-Darstellung die ermittelte Position und Orientierung des Endoskops angezeigt werden, ggf. zusammen mit aufgenommenen endoskopischen Bilddaten. Ferner kann eine Aufzeichnung der ermittelten Positionen und Orientierungen erfolgen sowie eine Anzeige der bereits erreichten Positionen und/oder Orientierungen des Endoskops.

Das Verfahren dient insbesondere zur Unterstützung eines Anwenders bei der endoskopischen Navigation, d.h. bei der gezielten Bewegung des Endoskops innerhalb des Hohlraums bei einer endoskopischen Untersuchung oder einem endoskopischen Eingriff. Das Verfahren ist insbesondere zur Unterstützung bei einer medizinischen endoskopischen Navigation in einem körperinneren Hohlraum eines menschlichen oder tierischen Körpers vorgesehen, kann aber auch bei nichtmedizinischen Anwendungen eingesetzt werden, etwa bei der Untersuchung von Hohlräumen in technischen Objekten.

Dadurch, dass eine auf das Endoskop einwirkende Beschleunigung, eine Drehbewegung des Endoskops und eine Einführbewegung des Endoskops erfasst werden und eine Position und eine Orientierung des Endoskops, insbesondere des distalen Endbereichs des Schafts des Endoskops, aufgrund der erfassten Werte der Beschleunigung und der Winkelgeschwindigkeit unter Berücksichtigung der erfassten Einführbewegung des Endoskops ermittelt werden, wird es ermöglicht, mit einfachen Mitteln und insbesondere mit erhöhter Genauigkeit eine Position und eine Orientierung des Endoskops bzw. der Endoskopspitze zu ermitteln. Erfindungsgemäß ist erkannt worden, dass die Ungenauigkeiten der Bestimmung der Position und der Orientierung des Endoskops verringert werden können, wenn die Einführbewegung bei der Einführung des Endoskops in den Hohlraum ermittelt wird, woraus insbesondere eine Einführlänge des Endoskops in den Hohlraum bestimmt werden kann. Da die Position der Zugangsöffnung, mit der die Einführvorrichtung verbunden ist, in der Regel relativ zu dem beobachteten Hohlraum weitgehend unveränderlich ist, können hierdurch Daten zur Ermittlung der Lage des Endoskops erfasst werden, die von Ungenauigkeiten des Beschleunigungssensors und des Drehratensensors, insbesondere von einer Drift der genannten Sensoren, unabhängig sind. Dadurch, dass die unter Verwendung der Signale des mindestens einen Beschleunigungs- und des mindestens einen Drehratensensors ermittelte Position und Orientierung des distalen Endbereichs des Endoskops mit den vom Einführsensor erfassten Daten, insbesondere mit einer aus den Daten des Einführsensors ermittelten Einführlänge, korrigiert werden können, ist eine Verbesserung der Genauigkeit der Bestimmung der Position und/oder der Orientierung des Endoskops, insbesondere des distalen Endbereichs des Schafts des Endoskops, möglich.

Vorzugsweise werden mit dem mindestens einen Einführsensor sowohl eine Längsbewegung als auch eine Drehbewegung des Schafts des Endoskops relativ zur Einführvorrichtung erfasst. Hierdurch ist sowohl eine Einführlänge beim Einführen des Endoskopschafts in den Hohlraum bzw. beim Herausziehen des Schafts aus dem Hohlraum bestimmbar als auch eine Rotation des Schafts um seine Längsachse, die auch bei unveränderter Einführlänge stattfinden kann. Dadurch, dass sowohl die Längs- als auch die Drehbewegung erfasst und bei der Ermittlung der Position und Orientierung berücksichtigt werden, ist eine besonders hohe Genauigkeit bei der Ermittlung der Position und der Orientierung des Endoskops bzw. des distalen Endbereichs des Endoskops innerhalb des Hohlraums erzielbar.

Weiterhin ist es bevorzugt, dass mit mindestens einem dem Endoskop zugeordneten, insbesondere in dem Endoskop aufgenommenen, Magnetfeldsensor (Magnetometer) eine Position und/oder Orientierung des Endoskops relativ zu einem externen Magnetfeld erfasst werden. Ein derartiger Magnetfeldsensor kann zum Zusammenwirken mit einem elektromagnetischen Navigationssystem, das das externe Magnetfeld erzeugt, oder auch zur Erfassung einer Richtung relativ zum Erdmagnetfeld ausgebildet sein. Die Berücksichtigung des Signals des Magnetfeldsensors bei der Ermittlung der Position und/oder Orientierung des Endoskops ermöglicht eine weitere Steigerung der Genauigkeit der Erfassung der Position und Orientierung des Endoskops.

Gemäß einer besonders bevorzugten Ausführungsform der Erfindung wird mit einem elektronischen Bildaufnehmer ein endoskopisches Bild eines Objektfelds innerhalb des Hohlraums aufgenommen; der elektronische Bildaufnehmer ist hierfür insbesondere in dem Endoskop aufgenommen oder an dieses angekoppelt. Anhand des aufgenommenen endoskopischen Bildes wird eine bildbasierte Bewegungserkennung durchgeführt, deren Ergebnisse bei der Ermittlung der Position und Orientierung des Endoskops berücksichtigt werden. Methoden zur bildbasierten Bewegungserkennung sind hier beispielsweise die Berechnung des optischen Flusses oder die Erkennung und Nachverfolgung markanter Strukturmerkmale. Insbesondere kann die Steuerungseinrichtung auch zur bildbasierten Bewegungserkennung eingerichtet sein. Gemäß diesem Aspekt der Erfindung wird ausgenutzt, dass das endoskopische Bild weitere Informationen enthält, die zur Unterstützung der Navigation genutzt werden können. Der optische Fluss und/oder die Bewegungsinformation aus der Nachverfolgung markanter Strukturmerkmale, die jeweils die Bewegungen des Bildinhalts im aufgenommenen endoskopischen Bild erfassen und wobei jedem Pixel bzw. jedem Strukturmerkmal ein Geschwindigkeitsvektor zugeordnet werden kann, können aus dem zeitlich veränderlichen endoskopischen Bild in an sich bekannter Weise ermittelt werden und ermöglichen eine von den Signalen des mindestens einen Beschleunigungssensors, des mindestens einen Drehratensensors und des mindestens einen Einführsensors unabhängige Erfassung der Bewegung des distalen Endbereichs des Endoskops relativ zum beobachteten Hohlraum. Die auf diese Weise gewonnenen Informationen über die lineare und/oder Winkelgeschwindigkeit des Endoskops können mit den Signalen des mindestens einen Beschleunigungssensors, des mindestens einen Drehratensensors und des mindestens einen Einführsensors sowie der daraus ermittelten Position und Orientierung des Endoskops korreliert bzw. zur Korrektur der betreffenden Daten genutzt werden. Hierdurch ist eine weitere Steigerung der Genauigkeit der Bestimmung der Position und Orientierung des Endoskops erzielbar.

Vorzugsweise wird die aus dem endoskopischen Bild ermittelte bildbasierte Bewegungserkennung genutzt, um einen Ruhezustand der Bewegung des Endoskops zu detektieren. Insbesondere wird ein Ruhezustand detektiert, wenn eine aus der bildbasierten Bewegungserkennung ermittelte Geschwindigkeit, aus der auf eine lineare und/oder Winkelgeschwindigkeit des Endoskops, insbesondere des distalen Endbereichs des Endoskops, geschlossen werden kann, gleich Null ist oder einen vorgebbaren Grenzwert unterschreitet. In diesem Fall kann davon ausgegangen werden, dass sich das Endoskop relativ zum Hohlraum in Ruhe befindet und die Position und Orientierung des Endoskops sich daher nicht verändern. Vorzugsweise werden zur Detektion des Ruhezustands auch die Signale des mindestens einen Drehratensensors und des mindestens einen Einführsensors herangezogen und der Ruhezustand dann detektiert, wenn die Signale der genannten Sensoren keine oder eine unterhalb eines jeweiligen Schwellwerts liegende Winkelgeschwindigkeit bzw. Einführgeschwindigkeit anzeigen. Weiter vorzugsweise kann zur Detektierung des Ruhezustands auch das Signal des mindestens einen Beschleunigungssensors ausgewertet werden und als zusätzliche Bedingung für die Erkennung des Ruhezustands dienen, dass der Beschleunigungssensor eine Beschleunigung erfasst, deren Betrag nicht oder nur um weniger als ein entsprechender Schwellwert von der Erdbeschleunigung abweicht. Die Detektion des Ruhezustands kann beispielsweise eine Voraussetzung für die Speicherung des aufgenommenen endoskopischen Bilds oder für die Durchführbarkeit bestimmter chirurgischer Manipulationen sein und einem Anwender angezeigt werden. Umgekehrt kann eine Beendigung des Ruhezustands dadurch detektierbar sein, dass die aus der bildbasierten Bewegungserkennung ermittelte Geschwindigkeit und/oder die Signale des mindestens einen Drehratensensors und/oder des mindestens einen Einführsensors über einen jeweiligen Schwellwert ansteigen bzw. das Signal des mindestens einen Beschleunigungssensors eine von der Erdbeschleunigung abweichende Beschleunigung anzeigt. Hierdurch kann die Sicherheit bei endoskopischen Eingriffen verbessert werden.

Vorzugsweise wird bei Detektion des Ruhezustands eine Kalibrierung des mindestens einen Beschleunigungssensors und/oder des mindestens einen Drehratensensors durchgeführt. Hierbei wird ausgenutzt, dass im Ruhezustand davon ausgegangen werden kann, dass sowohl die Geschwindigkeit einer linearen Bewegung als auch eine Winkelgeschwindigkeit Null oder nahezu Null sind, so dass die durch eine eventuelle Drift verursachten Abweichungen der aus den Signalen der genannten Sensoren ermittelten linearen Geschwindigkeit bzw. Winkel korrigiert werden können. Sofern bei einer solchen Ruhelage somit eine wesentlich von Null verschiedene lineare oder Winkelgeschwindigkeit ermittelt wird, so wird diese als durch eine Drift bedingt interpretiert und von den weiteren ermittelten Werten abgezogen. Besonders vorteilhaft ist es, die bildbasierte Bewegungserkennung mit den Daten der Inertialsensoren derart zu verknüpfen, dass eine Kalibrierung dann durchgeführt wird, wenn der Beschleunigungssensor sowie vorzugsweise auch der Drehratensensor keine Bewegung detektieren und zudem im endoskopischen Bild keine Drehbewegung erkennbar ist. Auf diese Weise kann während kurzeitiger Ruhezustände des Endoskops eine Rekalibrierung der Sensoren erfolgen, wobei die Rekalibrierung automatisch oder manuell erfolgen kann. Insbesondere kann aus den bei der Rekalibrierung jeweils abgezogenen Werten, die als Drift interpretiert werden, und der Zeitspanne zwischen den Rekalibrierungen auf einen maximal möglichen Winkelfehler geschlossen werden. Bei Überschreiten eines für die jeweilige Anwendung kritischen Wertes kann ein Anwender hierauf hingewiesen werden; es kann dann beispielsweise dadurch eine manuelle Kalibrierung erfolgen, dass das Endoskop in einen definierten Winkelbereich bewegt wird.

Weiter vorzugsweise kann auch das Signal des mindestens einen Einführsensors, insbesondere in dem Fall, dass dieser eine Vorschubgeschwindigkeit und/oder eine Winkelgeschwindigkeit erfasst, bei Erkennung des Ruhezustands kalibriert werden. Ferner kann in dem Fall, dass das Endoskop einen Magnetfeldsensor umfasst, in vorteilhafter Weise der detektierte Ruhezustand genutzt werden, um zeitliche Änderungen des Magnetfelds zu erkennen und so die Zuverlässigkeit des Magnetfeldsensors zu bewerten. Hierdurch ist eine weitere Steigerung der Genauigkeit der Bestimmung der Position und der Orientierung des Endoskops erreichbar.

In besonders vorteilhafter Weise kann es vorgesehen sein, dass im aufgenommenen endoskopischen Bild mindestens ein markantes Strukturmerkmal erkannt wird, insbesondere automatisch detektiert wird, und die Lage des Strukturmerkmals im Bild mit den parallel hierzu ermittelten Navigationsdaten, d.h. mit der dazugehörigen Position und Orientierung des Endoskops, bei einem ersten detektierten Ruhezustand gespeichert wird. Wird nachfolgend ein weiterer Ruhezustand erkannt, so wird das Strukturmerkmal erneut detektiert und eine Kalibrierung des mindestens einen Beschleunigungssensors und/oder des mindestens einen Drehratensensors aufgrund der aktuellen Lage des Strukturmerkmals im Bild und der gespeicherten Lage des Strukturmerkmals sowie der zuvor gespeicherten zugehörigen Position und Orientierung des Endoskops durchgeführt. Wenn bei detektierter Ruhelage eine Rekalibrierung erfolgt, kann auf diese Weise die entsprechende Lage des Endoskops im Patienten direkt erfassbar sein, da die vorherige Lage der Strukturmerkmale sowie die Position und Orientierung des Endoskops bekannt sind. Hierdurch kann eine Rekalibrierung mit einer besonders hohen Genauigkeit erfolgen, die insbesondere von der verstrichenen Zeit und einer eventuellen Drift der Sensoren unabhängig ist, da ein bereits akkumulierter Winkelfehler wieder zurückgesetzt werden kann.

Weiterhin ist es bevorzugt, dass aufgrund einer Detektion des Ruhezustands des Endoskops eine Aufnahme bzw. Akquisition und/oder Übertragung und/oder Speicherung des endoskopischen Bilds unterbrochen wird. Da im Ruhezustand keine wesentliche Veränderung des aufgenommenen endoskopischen Bilds erfolgt, kann hierdurch eine unnötige Inanspruchnahme von Übertragungskanälen, Prozessorleistung und/oder Speicherplatz vermieden werden. Nach einer vorgebbaren Zeitspanne oder auch wenn etwa aufgrund eines Signals des Beschleunigungssensors, des Drehratensensors und/oder des Einführsensors eine Beendigung des Ruhezustands detektiert wird, kann die Akquisition, Übertragung und Speicherung des endoskopischen Bilds wieder aufgenommen werden.

In vorteilhafter Weise kann weiterhin vorgesehen sein, dass das Endoskop eine Distanzmesseinrichtung umfasst, wodurch etwa mittels strukturierter Beleuchtung oder Laufzeitmessung (Time of Flight, TOF) ein Abstand des Endoskops, insbesondere der Endoskopspitze, zur Wand des Hohlraums ermittelt wird. Mit Hilfe der derart ermittelten Abstandsdaten kann ein 3D-Modell einer Umgebung, insbesondere des Hohlraums, in den das Endoskop eingeführt ist, erstellt werden. Ferner kann insbesondere in dem Fall, dass die Bewegung des Hohlraums vernachlässigbar gering ist, die aufgrund der erfassten Beschleunigung, Drehbewegung und Einführbewegung ermittelte Position und Orientierung des Endoskops mit Hilfe der Abstandsdaten überprüft und ggf. korrigiert werden.

Ein erfindungsgemäßes Endoskopsystem umfasst ein Endoskop mit einem durch eine Zugangsöffnung, die insbesondere eine natürlich oder eine künstlich geschaffene Körperöffnung sein kann, in einen Hohlraum, insbesondere in einen körperinneren Hohlraum eines Patienten, einführbaren langerstreckten Schaft. In dem Schaft ist eine Beobachtungsoptik zur Erzeugung eines endoskopischen Bilds eines Objektfelds in dem Hohlraum aufgenommen. Die Beobachtungsoptik umfasst insbesondere ein Endoskopobjektiv, das in einem distalen Endbereich des Schafts angeordnet ist und das ein erstes Zwischenbild der aufgenommenen Szene in dem Hohlraum erzeugt. Die Beobachtungsoptik kann außerdem einen Bildweiterleiter zur Weiterleitung des ersten Zwischenbilds zum proximalen Endbereich des Schafts umfassen, der durch ein geordnetes Glasfaserbündel (Bildleiter) oder, bei einem starren Endoskop, durch hintereinander angeordnete Relaislinsensysteme gebildet werden kann. Weiterhin kann das Endoskop einen elektronischen Bildaufnehmer zur Aufnahme des von der Beobachtungsoptik erzeugten endoskopischen Bilds aufweisen, beispielsweise ein CCD (Charge Coupled Device). Der elektronische Bildaufnehmer kann im distalen Endbereich des Schafts angeordnet sein und beispielsweise das vom Endoskopobjektiv erzeugte erste Zwischenbild aufnehmen oder auch etwa in einem am proximalen Ende des Schafts angeordneten Endoskopkopf angeordnet sein und das von einem Bildweiterleiter dorthin weitergeleitete Bild aufnehmen. Ferner ist es möglich, dass der elektronische Bildaufnehmer Teil einer im proximalen Endbereich des Endoskops an dieses anschließbaren endoskopischen Videokamera ist. Das vom elektronischen Bildaufnehmer aufgenommene Bild kann von einer Auswertungs- und Anzeigeeinrichtung ausgewertet, auf einem Bildschirm für einen Anwender dargestellt und/oder beispielsweise für Dokumentationszwecke gespeichert werden. Der proximale Endbereich des Endoskops kann als Endoskopkopf bzw. als Handgriff ausgebildet sein und weitere Anschlüsse, etwa für die Einkopplung von Beleuchtungslicht und/oder für Spül- und Saugleitungen und/oder Versorgungsleitungen zur Ansteuerung und Signalübertragung eines im Endoskop aufgenommenen elektronischen Bildaufnehmers aufweisen.

Das Endoskop umfasst erfindungsgemäß mindestens einen Beschleunigungssensor und mindestens einen Drehratensensor, die zur Erfassung einer auf das Endoskop einwirkenden Beschleunigung und/oder Schwerkraftwirkung bzw. einer Drehbewegung des Endoskops ausgebildet und angeordnet sind. Der mindestens eine Beschleunigungssensor und der mindestens eine Drehratensensor können in an sich bekannter Weise in miniaturisierter Form ausgebildet sein, so dass diese in den begrenzten Bauraum, der in einem Endoskop zur Verfügung steht, integriert werden können. Vorzugsweise sind der mindestens eine Beschleunigungssensor und der mindestens eine Drehratensensor als MEMS (Micro Electro Mechanical System) - Sensor ausgebildet.

Weiterhin umfasst das erfindungsgemäße Endoskopsystem eine endoskopische Einführvorrichtung, die in einer festen Beziehung zur Zugangsöffnung, durch welche der Schaft des Endoskops in den Hohlraum einführbar ist, gehalten werden kann. Die endoskopische Einführvorrichtung definiert insbesondere eine als Durchgangsöffnung ausgebildete Einführöffnung zum Einführen des Endoskops in einen körperinneren Hohlraum und ist hierfür mit der natürlichen oder künstlich geschaffenen Körperöffnung verbindbar bzw. in diese einsetzbar. Die endoskopische Einführvorrichtung kann insbesondere als Trokar bzw. Trokartubus ausgebildet sein. Erfindungsgemäß weist die endoskopische Einführvorrichtung mindestens einen Einführsensor auf, der zum Erfassen einer Einführbewegung des Schafts des Endoskops in den Hohlraum ausgebildet und angeordnet ist. Hierfür kann der Einführsensor insbesondere an der durch die Einführvorrichtung gebildeten Einführöffnung angeordnet sein. Vorzugsweise ist der Einführsensor auch zur Erfassung einer Herausziehbewegung beim Entnehmen des Schafts des Endoskops aus dem Hohlraum sowie zur Erfassung von weiteren Bewegungen des Schafts, insbesondere Drehbewegungen, ausgebildet.

Das erfindungsgemäße Endoskopsystem umfasst ferner eine Steuerungseinrichtung, die zur Ermittlung einer Position und einer Orientierung des distalen Endbereichs des Schafts des Endoskops eingerichtet ist, wobei die Position und die Orientierung aufgrund der Signale des mindestens einen Beschleunigungssensors, des mindestens einen Drehratensensors und des mindestens einen Einführsensors ermittelt werden. Die Steuerungseinrichtung ist zur Erfassung der Signale der betreffenden Sensoren mit diesen verbunden und umfasst Prozessormittel zur Ermittlung der Position und Orientierung des distalen Endbereichs des Schafts des Endoskops. Die Steuerungseinrichtung kann weiterhin zur Anzeige der Position und der Orientierung des distalen Endbereichs des Schafts des Endoskops in grafischer oder nummerischer Form eingerichtet sein und hierfür mit einer entsprechenden Anzeigeeinrichtung verbindbar sein oder eine solche umfassen.

Dadurch, dass das Endoskop mindestens einen Beschleunigungssensor und mindestens einen Drehratensensor umfasst, dass die Einführvorrichtung mindestens einen Einführsensor umfasst und dass die Steuerungseinrichtung zur Ermittlung einer Position und einer Orientierung des distalen Endbereichs des Schafts des Endoskops aufgrund der Signale des mindestens einen Beschleunigungssensors, des mindestens einen Drehratensensors und des mindestens einen Einführsensors eingerichtet ist, wird ein Endoskopsystem geschaffen, das auf einfache und kostengünstige Weise eine Bestimmung der Position und Orientierung eines Endoskops in einem Hohlraum ermöglicht.

Vorzugsweise ist der Einführsensor als ein optischer Sensor ausgebildet, der die Bewegung einer Oberfläche des Schafts relativ zur Einführvorrichtung, insbesondere relativ zur Einführöffnung, erfasst. Ein solcher optischer Sensor kann beispielsweise eine Beleuchtungseinrichtung und eine Bilderfassungseinrichtung sowie eine Bildanalyseeinrichtung umfassen, wobei aus der Analyse des von der Bilderfassungseinrichtung aufgenommenen, aufgrund der Bewegung des Schafts relativ zur Einführvorrichtung zeitlich veränderlichen Bilds ein Betrag und eine Richtung der Bewegung der Oberfläche des Schafts relativ zur Einführvorrichtung ermittelt werden können. Ein derartiger Sensor wird beispielsweise in einer optischen Computermaus verwendet. Auf diese Weise können die Geschwindigkeit einer Längsbewegung und eine Rotationsgeschwindigkeit des Schafts ermittelt werden. In der Regel ist für diesen Zweck die Rauigkeit der Oberfläche des Schafts ausreichend; es können aber auch Marken auf der Oberfläche des Schafts angebracht sein. Die Marken können gemäß einer besonderen Ausführungsform derart ausgebildet sein, dass eine absolute Erfassung der Einführlänge und der Drehstellung des Schafts relativ zur Einführvorrichtung erfolgen kann.

Gemäß einer bevorzugten Ausführungsform der Erfindung ist das Endoskop als starres Endoskop ausgebildet. Da der distale und der proximale Endbereich des Endoskops starr miteinander verbunden sind, können der mindestens eine Beschleunigungssensor und der mindestens eine Drehratensensor sowohl im proximalen Endbereich des Endoskops wie im distalen Endbereich des Endoskops oder an anderen Positionen im Endoskop angeordnet sein; die Sensoren können auch an unterschiedlichen Positionen im Endoskop angeordnet sein. Der mindestens eine Drehratensensor erfasst eine Drehrate des Endoskops und der mindestens eine Beschleunigungssensor eine Beschleunigung desjenigen Bereichs des Endoskops, worin der Beschleunigungssensor aufgenommen ist; eine Beschleunigung des distalen Endbereichs des Endoskops, worin das Endoskopobjektiv zur Erzeugung eines endoskopischen Bilds angeordnet ist, kann aus dem Signal des Beschleunigungssensors und dem Signal des mindestens einen Drehratensensors unter Berücksichtigung des festen Abstands zwischen dem Beschleunigungs- und dem Drehratensensor errechnet werden. Hierdurch ist auf besonders einfache Weise eine Erfassung der Position und der Orientierung der Endoskopspitze möglich. Insbesondere bestimmt sich bei einem starren Endoskop die relative Raumposition der Endoskopspitze zur Einführvorrichtung direkt aus der Messung der Einführlänge und den drei Winkeln im Raum.

Gemäß einer weiteren bevorzugten Ausführungsform der Erfindung ist das Endoskop als flexibles Endoskop ausgebildet, wobei der mindestens eine Beschleunigungssensor und der mindestens eine Drehratensensor vorzugsweise im distalen Endbereich des Endoskopschafts angeordnet sind. Auch wenn der distale Endbereich des Endoskops hierbei nicht in einer starren Beziehung zum proximalen Endbereich steht, ist dennoch in der Regel durch Auswertung des Signals des Einführsensors eine Verbesserung der Genauigkeit der Bestimmung der Position und Orientierung des distalen Endbereichs des Endoskopschafts möglich. Eine weitere Steigerung der Genauigkeit lässt sich in diesem Fall dadurch erzielen, dass die mit dem Einführsensor ermittelte Einführ- bzw. Herausziehgeschwindigkeit, d.h. die zeitliche Veränderung der Einführlänge, in der jeweiligen, zeitlich veränderlichen räumlichen Orientierung der Endoskopspitze ermittelt und zeitlich aufsummiert bzw. integriert wird, um die aus den Daten der Inertialsensoren ermittelte Position der Endoskopspitze mit den Daten des Einführsensors zu korrigieren.

Das erfindungsgemäße Endoskopsystem ist insbesondere zur Durchführung des oben beschriebenen Verfahrens zur Bestimmung der Position und Orientierung des Endoskops ausgebildet. Hierzu kann beispielsweise wie oben beschrieben das Endoskop zusätzlich einen Magnetfeldsensor zur Erfassung einer Position und/oder Orientierung des Endoskops relativ zu einem externen Magnetfeld und/oder einen elektronischen Bildaufnehmer zur Aufnahme eines endoskopischen Bilds für eine bildbasierte Bewegungserkennung umfassen, wobei die Steuerungseinrichtung jeweils zur Ermittlung der Position und Orientierung des Endoskops aufgrund der erfassten Daten in der oben beschriebenen Weise eingerichtet ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:

Fig. 1 ein Ausführungsbeispiel eines erfindungsgemäßen Endoskopsystems in schematischer Darstellung;

Fig. 2 ein erstes Ausführungsbeispiel einer Sensoranordnung bei einem Endoskopsystem gemäß Fig. 1 mit einem starren Endoskop;

Fig. 3 ein zweites Ausführungsbeispiel einer Sensoranordnung bei einem Endoskopsystem gemäß Fig. 1 mit einem starren Endoskop;

Fig. 4 ein Ausführungsbeispiel einer Sensoranordnung bei einem erfindungsgemäßen Endoskopsystem mit einem flexiblen Endoskop;

Fig. 5 ein Beispiel für eine Anzeige der erfindungsgemäß ermittelten Position und Orientierung eines Endoskops für einen Anwender;

Fig. 6 ein Beispiel für eine Anzeige der bereits abgebildeten Bereiche eines körperinneren Hohlraums unter Verwendung der erfindungsgemäß ermittelten Position und Orientierung eines Endoskops.

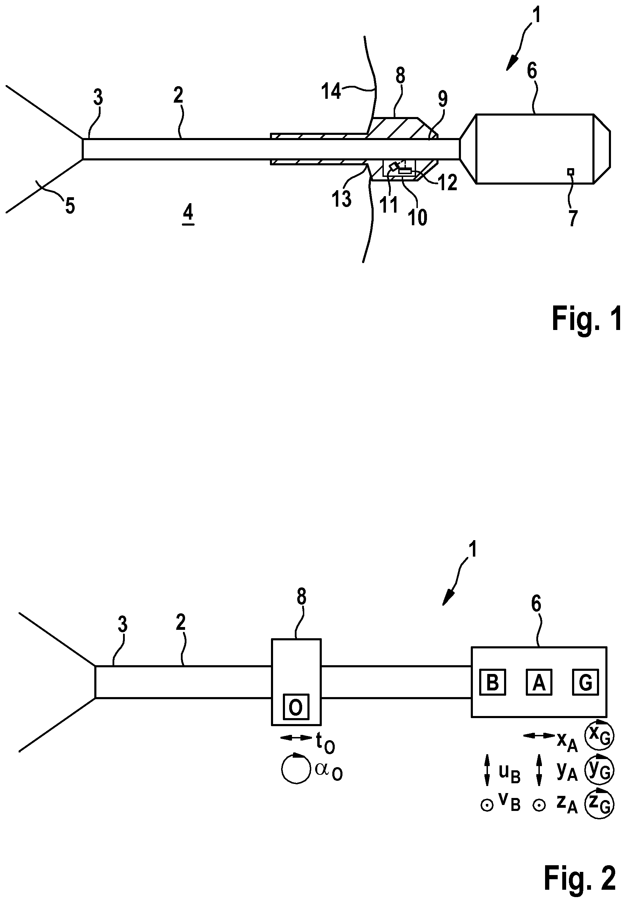

In Fig. 1 ist in schematischer Form ein Ausführungsbeispiel eines erfindungsgemäßen Endoskopsystems dargestellt, wobei die Steuerungseinrichtung sowie Versorgungs- und Verbindungsleitungen nicht gezeigt sind. Das Endoskop 1 umfasst einen langerstreckten zylindrischen Schaft 2, der starr, halbstarr oder flexibel ausgebildet sein kann. Im distalen Endbereich 3 des Schafts 2 ist ein Endoskopobjektiv angeordnet, mit dem ein Bild einer Szene in einem Hohlraum 4, insbesondere eines Objektfelds an der Innenwand des Hohlraums, erzeugt werden kann. Ein Bereich des Hohlraums 4, auf den das Endoskopobjektiv gerichtet ist, wird hierfür mit aus dem distalen Ende des Endoskops 1 austretendem Beleuchtungslicht beleuchtet, das in Fig. 1 als Lichtkegel 5 dargestellt ist. In Fig. 1 ist ein als Geradeausblickoptik ausgebildetes Endoskop 1 gezeigt; es ist auch möglich, dass die Blickrichtung des Endoskopobjektivs und der Lichtkegel 5 des Beleuchtungslichts seitlich bzw. abgewinkelt zur Längsachse des Schafts 2 gerichtet sind. Innerhalb des Schafts 2 verlaufen Lichtleiter oder elektrische Leitungen zur Weiterleitung von Beleuchtungslicht vom proximalen zum distalen Endbereich 3 des Schafts 2 bzw. zur Versorgung einer im distalen Endbereich 3 angeordneten Lichtquelle. Ferner ist im Schaft 2 ein Bildweiterleiter zur Weiterleitung des vom Endoskopobjektiv erzeugten Bilds der Szene zum proximalen Ende des Schafts 2 angeordnet, oder, wenn im distalen Endbereich 3 ein elektronischer Bildaufnehmer zur Aufnahme des Bilds aufgenommen ist, elektrische Leitungen zur Versorgung des Bildaufnehmers und zur Übertragung des Signals des Bildaufnehmers zum proximalen Endbereich. Am proximalen Ende des Schafts 2 ist ein Endoskopkopf 6 angeordnet, der einen elektronischen Bildaufnehmer sowie nicht dargestellte Versorgungsanschlüsse umfassen kann. Weiter weist der Endoskopkopf 6 eine symbolisch dargestellte Inertialsensoranordnung mit einem Beschleunigungs- und einem Drehratensensor auf, die als MEMS-Baustein 7 ausgebildet ist. Derartige MEMS-Sensorbausteine sind kompakt mit Maßen von nur ca. 3mm x 3mm x 1mm, sind robust gegenüber mechanischen Belastungen und sind kostengünstig verfügbar.

Das Endoskopsystem umfasst ferner einen Trokartubus 8, der eine Einführöffnung 9 bildet, durch die der Schaft 2 des Endoskops 1 geschoben werden kann. An der Einführöffnung 9 ist ein Einführsensor angeordnet, der als optischer Sensor 10 ausgebildet ist. Wie in Fig. 1 gezeigt, ist der optische Sensor 10 nach Art des Sensors einer optischen Computermaus ausgebildet und umfasst eine LED 11 zur Beleuchtung der Oberfläche des Schafts 2, einen Bildsensor 12 und eine nicht dargestellte Bildanalyseeinrichtung, wodurch eine Verschiebung und eine Rotation des Schafts 2 in der Einführöffnung 9 erfassbar sind. Geeignete optische Computermaus-Sensoren sind kompakt (mit weniger als 1cm Durchmesser) und sind kostengünstig verfügbar.

Wie in Fig. 1 angedeutet, ist der Trokartubus 8 in eine Körperöffnung 13 eingesetzt und in dieser gehalten, die durch eine Inzision in der Bauchdecke 12 geschaffen worden ist, die aber auch beispielsweise eine natürliche Körperöffnung sein kann. Durch die Einführöffnung 9 des Trokartubus 8 ist der Schaft 2 des Endoskops 1 in den hierdurch zugänglich gemachten oder durch Insufflation geschaffenen Hohlraum 4 eingeschoben worden. Auf diese Weise kann ein endoskopisches Bild eines Objektfelds aufgenommen werden. Um die Navigation innerhalb des Hohlraums 4 zu ermöglichen bzw. zu erleichtern, werden die Signale der Inertialsensoranordnung, die mindestens einen Beschleunigungs- und einen Drehratensensor umfasst, des Einführsensors und eines zur Aufnahme des endoskopischen Bilds vorgesehenen Bildaufnehmers sowie ggf. eines Magnetfeldsensors erfasst und ausgewertet. Hierfür ist eine in Fig. 1 nicht gezeigte Steuerungseinrichtung vorgesehen, die mit den betreffenden Sensoren verbunden und zur entsprechenden Auswertung der Signale eingerichtet ist.

Wie in Fig. 2 schematisch dargestellt, sind gemäß einem ersten Ausführungsbeispiel einer Sensoranordnung bei einem erfindungsgemäßen Endoskopsystem, wobei das Endoskop 1 als starres Endoskop ausgebildet ist, sowohl der Bildaufnehmer B zur Aufnahme des endoskopischen Bilds als auch der Beschleunigungssensor (Accelerometer A) und der Drehratensensor (Gyroskop G) im Endoskopkopf 6 des Endoskops 1 angeordnet. Der Bildaufnehmer B liefert Bilddaten, aus denen über eine bildbasierte Bewegungserkennung, beispielsweise durch Ermittlung des optischen Flusses und/oder durch Nachverfolgung von Strukturmerkmalen, eine vertikale und eine horizontale Geschwindigkeit $u_B$, $v_B$ der beobachteten Szene ermittelt werden können. Das Accelerometer A, das als 3D-Accelerometer ausgebildet ist, erfasst die Beschleunigungen $x_A$, $y_A$, $z_A$ in drei zueinander orthogonalen Raumrichtungen. Das Gyroskop G ist ein 3D-Gyroskop und liefert die Drehgeschwindigkeiten $x_G$, $y_G$, $z_G$ um die drei Raumachsen. Aus den Daten des optischen Sensors O an der Einführöffnung können die Einführlänge $t_0$ und der Drehwinkel $\alpha_0$ einer Drehung um die Längsachse des Schafts 2 ermittelt werden.

Das Accelerometer A und das Gyroskop G liefern eine Beschleunigung bzw. eine Winkelgeschwindigkeit des Endoskops 1. Die Position und die Orientierung des Endoskops 1 im Raum können hieraus nur indirekt ermittelt werden. Insbesondere erhält man Winkel, die die Orientierung des Endoskops 1 angeben, nur dadurch, dass die zeitlich gemessenen Änderungen aufsummiert bzw. die gemessenen Winkelgeschwindigkeiten zeitlich integriert werden. Da sich dadurch auch die Messfehler addieren, kommt es zu einer zeitlich anwachsenden Abweichung zwischen dem wahren und dem mit dem Gyroskop G bestimmten Winkel, die auch als "Drift" bezeichnet wird. Da das Accelerometer A nur Neigungen relativ zur Richtung des Gravitationsfelds erfassen kann, können hierüber nur zwei Rotationsfreiheitsgrade erfasst und die Drift des Gyroskops nur unvollständig korrigiert werden. Die Neigung relativ zur Gravitationsrichtung kann auch zur Bildaufrichtung des aufgenommenen endoskopischen Bilds verwendet werden. Die Rotation um die Richtung des Gravitationsfelds, d.h. um eine vertikale Achse, kann hiermit nicht korrigiert werden. Eine Position im Raum kann aus den vom Accelerometer A gemessenen Beschleunigungen $x_A$, $y_A$, $z_A$ nach Abzug der Erdbeschleunigung durch zweifache zeitliche Integration ermittelt werden, wobei die Messabweichungen ebenfalls mit der Zeit zunehmen.

Bei der in Fig. 2 gezeigten Anordnung kann mit der aus der bildbasierten Bewegungserkennung ermittelten horizontalen und vertikalen Bewegung $u_B$, $v_B$ der beobachteten Szene im aufgenommenen endoskopischen Bild eine Rotation unabhängig vom Gyroskop G zumindest näherungsweise erfasst werden. So stellt beispielsweise dann, wenn das Endoskop 1 mit der Längsachse des Schafts 2 horizontal ausgerichtet ist, die horizontale Geschwindigkeit $u_B$ eine Rotation des Endoskops um die vertikale Achse dar. Wird aus der bildbasierten Bewegungserkennung ermittelt, dass

$$u_B = 0$$

ist, so kann in guter Näherung davon ausgegangen werden, dass die Winkelgeschwindigkeit um die vertikale Achse ebenfalls Null ist. Die vom Gyroskop G ermittelte entsprechende Drehgeschwindigkeit $z_G$ kann dann rekalibriert, d.h. auf Null gesetzt werden. Hierdurch wird die Genauigkeit der Bestimmung der Position und der Orientierung des distalen Endbereichs des Endoskops 1 verbessert. Weiter kann die aus der Beschleunigung $x_A$ ermittelte Bewegung des Endoskops in Längsrichtung des Schafts mit der Einführlänge $t_0$ korrigiert werden, die mit dem optischen Sensor O direkt oder auch durch zeitliche Integration einer Einführgeschwindigkeit ermittelt wird.

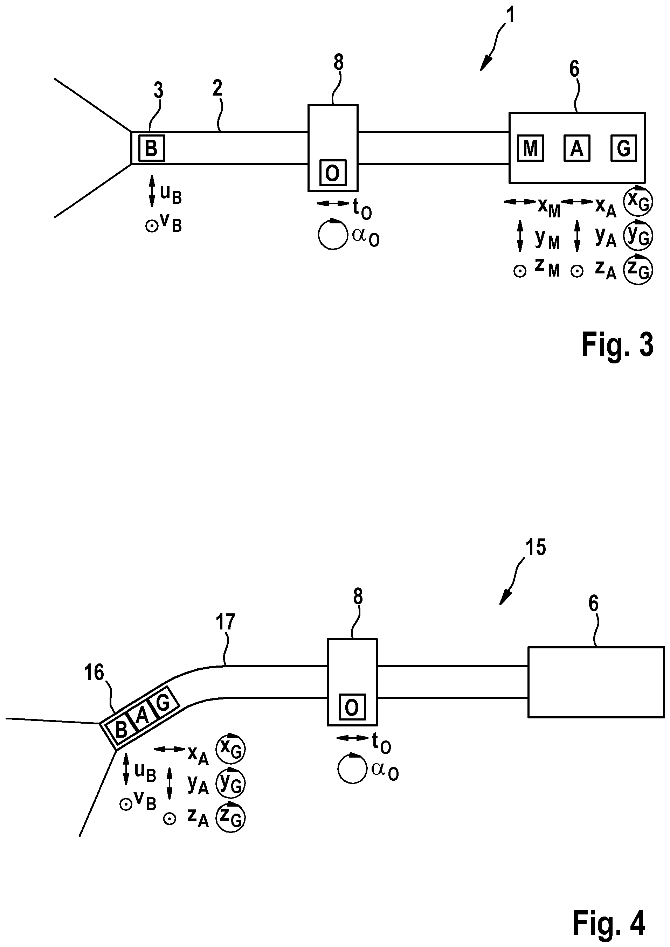

Wie in Fig. 3 dargestellt ist, kann der Bildaufnehmer B zur Erfassung des endoskopischen Bilds stattdessen im distalen Endbereich 3 des Schafts 2 angeordnet sein. Wie in Fig. 3 gezeigt, kann zusätzlich ein Magnetfeldsensor (3D-Magnetometer M) vorgesehen sein, der die Magnetfeldstärke $x_M$, $y_m$, $z_m$ in den drei Raumrichtungen liefert. Hieraus kann eine Neigung relativ zur Achse des Erdmagnetfelds bestimmt werden, d.h. zwei Rotationsfreiheitsgrade. Da diese in der Regel jedoch unterschiedlich sind zu den über das Accelerometer A bestimmten Neigungswinkeln zur Gravitationsrichtung, kann die vom Magnetometer M gelieferte Information prinzipiell genutzt werden, um gemeinsam mit dem Accelerometer A die drei Rotationsfreiheitsgrade im Raum zu bestimmen. Allerdings kann die Messung des Magnetometers M durch im klinischen Umfeld nicht auszuschließende Störungen des Magnetfeldes, etwa durch metallische Abschirmungen, ferromagnetische Objekte oder Abstrahlung von weiteren Geräten, gestört werden. Die vom Magnetometer gelieferten Messwerte der Magnetfeldstärke $x_M$, $y_M$, $z_M$ sind daher allein nicht ausreichend genau, können aber hilfreich sein, um die Informationen aus den Messungen des Accelerometers A und des Gyroskops G zu korrigieren bzw. die Genauigkeit der Positions- und Orientierungsbestimmung zu verbessern. Im Übrigen ist die in Fig. 3 dargestellte Sensoranordnung wie die in Fig. 2 gezeigte.

In Fig. 4 ist ein flexibles Endoskop 15 dargestellt, bei dem sowohl der Bildaufnehmer B als auch der Beschleunigungssensor (Accelerometer A) und der Drehratensensor (Gyroskop G) im distalen Endbereich 16 des flexiblen Schafts 17 angeordnet sind. Im Übrigen ist das Ausführungsbeispiel gemäß Fig. 4 wie die zuvor beschriebenen Ausführungsbeispiele ausgebildet. Dadurch, dass der Bildaufnehmer B, das Accelerometer A und das Gyroskop G im distalen Endbereich 16 des flexiblen Schafts 17 angeordnet sind, ist direkt eine Erfassung der auf den distalen Endbereich 16 einwirkenden Beschleunigungen $x_A$, $y_A$, $z_A$ in den drei Raumrichtungen sowie der Drehgeschwindigkeiten $x_G$, $y_G$, $z_G$ um die drei Raumachsen möglich. Die Einführlänge $t_0$ und der Drehwinkel $\alpha_0$, die mit dem optischen Sensor O des Trokartubus 8 ermittelt werden, können benutzt werden, um die Genauigkeit der Bestimmung der Position der Endoskopspitze, d.h. des distalen Endbereichs 16 des flexiblen Schafts 17, zu verbessern, wobei die Veränderung der Einführlänge mit der jeweiligen Orientierung des distalen Endbereichs 16 vektoriell aufsummiert wird.

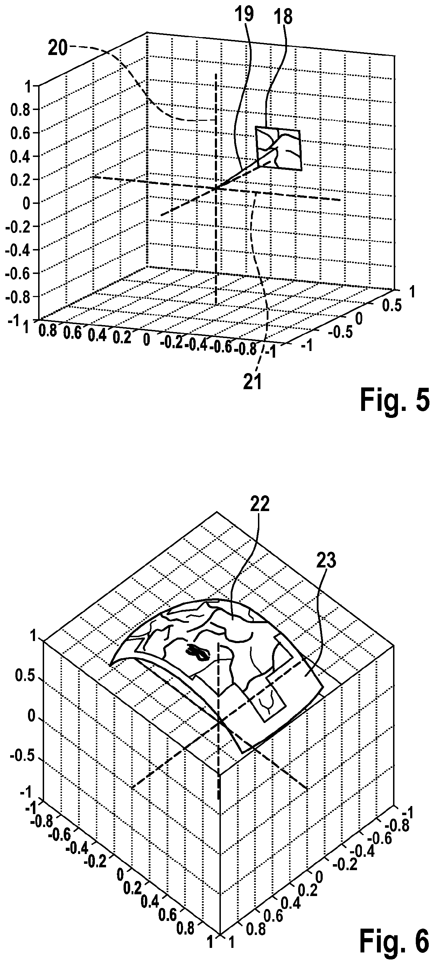

Das erfindungsgemäße Endoskopsystem kann beispielsweise bei einer Blasenspiegelung verwendet werden. Dabei kann einem Anwender nach Art einer 3D-Darstellung angezeigt werden, wo das Endoskop aktuell im Raum steht und in welche Raumrichtung das Endoskop schaut. Ein Beispiel für eine solche Darstellung ist in Fig. 5 gezeigt, wobei das endoskopische Bild 18 eines aktuell beobachteten Bereichs der Blasenwand zusammen mit der Längsachse 19 des Endoskops und ggf. weiteren Achsen 20, 21 eines mit dem Endoskop verbundenen Koordinatensystems dargestellt werden kann. Um über die Bestimmung der Position und Orientierung des Endoskops hinaus auch ein grobes 3D-Modell des eingesehenen Bereichs der Blasenwand zu erstellen, kann es ausreichen, dass einzelne Punkte des Bereichs mit der Endoskopspitze angefahren und die entsprechenden Positionen der Endoskopspitze gespeichert werden; alternativ kann in dem Fall, dass das Endoskop eine Distanzmesseinrichtung umfasst, ein Abstand des Endoskops bzw. der Endoskopspitze zu einzelnen Punkten des Bereichs der Blasenwand erfasst und hieraus die Positionen der Punkte ermittelt werden. Kann das eingesehene Volumen in der für die jeweilige Anwendung geforderten Genauigkeit mit einem geometrischen Modell angenähert werden, etwa einer Kugel für die Darstellung eingesehener Winkelbereiche in einer Blase, können solche einzelnen Punkte für die Approximation der Geometrie und für die Erstellung eines 3D-Modells ausreichen.

Weiterhin können kontinuierlich die ermittelte Position und die Orientierung der Endoskopspitze gespeichert werden. Auf einer Anzeigeeinrichtung können diese in einer Form dargestellt werden, dass für den Anwender des Endoskops sofort ersichtlich ist, welche Bereiche der Blase bereits endoskopisch untersucht bzw. abgebildet worden sind und welche nicht. Ein Beispiel für eine solche Darstellung ist in Fig. 6 gezeigt. Hierbei werden die aufgenommenen endoskopischen Bilder derart überlagert bzw. aneinander angefügt, dass die bereits untersuchten Winkelbereiche durch die jeweilige Bildinformation dargestellt werden (Bereich 22). Die noch nicht abgebildeten Winkelbereiche bleiben frei (Bereich 23). Diese Darstellung kann insbesondere nach Art einer 3D-Ansicht laufend angezeigt werden. Hierdurch wird eine vollständige Untersuchung der Blase erleichtert.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

Bezugszeichenliste

| 1 | Endoskop |
|---|---|
| 2 | Schaft |
| 3 | Distaler Endbereich |
| 4 | Hohlraum |
| 5 | Lichtkegel |
| 6 | Endoskopkopf |
| 7 | MEMS-Baustein |
| 8 | Trokartubus |
| 9 | Einführöffnung |
| 10 | Optischer Sensor |
| 11 | LED |
| 12 | Bildsensor |
| 13 | Zugangsöffnung |
| 14 | Bauchdecke |
| 15 | Flexibles Endoskop |
| 16 | Distaler Endbereich |
| 17 | Flexibler Schaft |
| 18 | Bild |
| 19 | Längsachse |
| 20 | Achse |
| 21 | Achse |
| 22 | Bereich |
| 23 | Bereich |
| A | Accelerometer |
| B | Bildaufnehmer |

G    Gyroskop
M    Magnetometer
O    Optischer Sensor

## Patentansprüche

1.  Endoskopsystem, umfassend ein Endoskop (1, 15) mit einem durch eine Zugangsöffnung (13) in einen Hohlraum (4) einführbaren langerstreckten Schaft (2, 17), wobei das Endoskop (1, 15) mindestens einen Beschleunigungssensor zur Erfassung einer auf das Endoskop (1, 15) einwirkenden Beschleunigung und mindestens einen Drehratensensor zur Erfassung einer Drehbewegung des Endoskops (1, 15) aufweist, eine endoskopische Einführvorrichtung, die in einer festen Beziehung zur Zugangsöffnung (13) haltbar ist, wobei die Einführvorrichtung mindestens einen Einführsensor zur Erfassung einer Einführbewegung des Schafts (2, 17) relativ zur Einführvorrichtung aufweist, und eine Steuerungseinrichtung, die zur Ermittlung einer Position und einer Orientierung des Endoskops (1, 15) aufgrund der erfassten Beschleunigung und Drehbewegung unter Berücksichtigung der Einführbewegung eingerichtet ist, wobei der Einführsensor als optischer Sensor (10, O) zur Erfassung einer Bewegung einer Oberfläche des Schafts (2, 17) relativ zur Einführvorrichtung ausgebildet ist, wobei mit dem mindestens einen Einführsensor sowohl eine Längsbewegung als auch eine Drehbewegung des Schafts (2, 17) des Endoskops (1, 15) relativ zur Einführrichtung erfassbar sind.

2.  Endoskopsystem nach Anspruch 1, **dadurch gekennzeichnet, dass** das Endoskop (1, 15) ein starres Endoskop (1) ist.

3.  Endoskopsystem nach Anspruch 1, **dadurch gekennzeichnet, dass** das Endoskop (1, 15) ein flexibles Endoskop (15) ist und dass der mindestens eine Beschleunigungssensor und der mindestens eine Drehratensensor im distalen Endbereich des Schafts (17) des flexiblen Endoskops (15) angeordnet sind.

## Claims

1.  An endoscopic system comprising an endoscope (1, 15) having an elongated shaft (2, 17) insertable into a cavity (4) through an access opening (13), wherein the endoscope (1, 15) has at least one acceleration sensor for capturing an acceleration acting on the endoscope (1, 15) and at least one angular rate sensor for capturing a rotational movement of the endoscope (1, 15), an endoscopic insertion device which is maintainable in a fixed relation to the access opening (13), wherein the insertion device has at least one insertion sensor for capturing an insertion movement of the shaft (2, 17) relative to the insertion device, and a control device which is configured for determining a position and an orientation of the endoscope (1, 15) on the basis of the captured acceleration and rotational movement taking into account the insertion movement, wherein the insertion sensor is implemented as an optical sensor (10, O) for capturing a movement of a surface of the shaft (2, 17) relative to the insertion device, wherein a longitudinal movement as well as a rotational movement of the shaft (2, 17) of the endoscope (1, 15) relative to the insertion device are capturable with the at least one insertion sensor.

2.  Endoscopic system according to claim 1, **characterized in that** the endoscope (1, 15) is a rigid endoscope (1).

3.  Endoscopic system according to claim 1 **characterized in that** the endoscope (1, 15) is a flexible endoscope (15) and that the at least one acceleration sensor and the at least one angular rate sensor are arranged within a distal end section of the shaft (17) of the flexible endoscope (15).

## Revendications

1.  Système d'endoscope, comprenant un endoscope (1, 15) avec une tige allongée (2, 17) pouvant être guidée à l'intérieur d'une cavité (4) à travers une ouverture d'accès (13), l'endoscope (1, 15) présentant au moins un capteur d'accélération pour détecter une accélération agissant sur l'endoscope (1, 15) et au moins un capteur de vitesse de rotation pour détecter un mouvement de rotation de l'endoscope (1, 15), un dispositif d'introduction endoscopique qui peut être retenu dans une relation fixe par rapport à l'ouverture d'accès (13), le dispositif d'introduction présentant au moins un capteur d'introduction pour détecter un mouvement d'introduction de la tige (2, 17) par rapport au dispositif d'introduction, et un dispositif de commande qui est prévu pour déterminer une position et une orientation de l'endoscope (1, 15) sur la base de l'accélération et du mouvement de rotation détectés en tenant compte du mouvement d'introduction, le capteur d'introduction étant réalisé sous forme de capteur optique (10, O) pour détecter un mouvement d'une surface de la tige (2, 17) par rapport au dispositif d'introduction, un mouvement longitudinal ainsi qu'un mouvement de rotation de la tige (2, 17) de l'endoscope (1, 15) par rapport à la direction d'introduction pouvant être détectés avec l'au moins un capteur d'introduction.

2.  Système d'endoscope selon la revendication 1, **ca-**

**ractérisé en ce que** l'endoscope (1, 15) est un endoscope rigide (1).

3.   Système d'endoscope selon la revendication 1, **caractérisé en ce que** l'endoscope (1, 15) est un endoscope flexible (15) et **en ce que** l'au moins un capteur d'accélération et l'au moins un capteur de vitesse de rotation sont disposés dans une région d'extrémité distale de la tige (17) de l'endoscope flexible (15).

**Fig. 1**

**Fig. 2**

Fig. 3

Fig. 4

**Fig. 5**

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6122538 A
- US 8419717 B2
- US 20110046637 A1
- US 20110032347 A1
- JP 2014230658 B
- JP 2014151102 B